# EUROPEAN PATENT SPECIFICATION

(11) **EP 2 129 686 B1**
(45) Date of publication and mention of the grant of the patent: **13.07.2016**
(21) Application number: 08716651.8
(22) Date of filing: 20.03.2008
(51) Int. Cl.: C07K 14/47, A61K 38/17, C07K 1/36

(54) **METHODS FOR INDUSTRIAL SCALE PRODUCTION OF THERAPEUTIC COMPLEMENT FACTOR H PREPARATIONS FROM HUMAN PLASMA**
VERFAHREN ZUR HERSTELLUNG IN INDUSTRIELLEM MASSSTAB VON THERAPEUTISCHEN KOMPLEMENTFAKTOR-H-PRÄPARATEN AUS MENSCHLICHEM PLASMA
PROCÉDÉS POUR LA PRODUCTION À L'ÉCHELLE INDUSTRIELLE DE PRÉPARATIONS THÉRAPEUTIQUES DU FACTEUR H DU COMPLÉMENT POUR LE PLASMA HUMAIN

(30) Priority: 20.03.2007 EP 07005655
(43) Date of publication of application: 09.12.2009
(73) Proprietor: CSL Behring GmbH, 35041 Marburg (DE)
(72) Inventor: LIEBING, Uwe, 35091 Cölbe (DE); KARGES, Hermann, 35041 Marburg (DE)
(74) Representative: Kalhammer, Georg
(86) International application number: PCT/EP2008/002238
(87) International publication number: WO 2008/113589

(56) References cited:
- EP-A1- 0 317 376
- EP-A1- 1 739 093
- EP-A2- 0 222 611
- WO-A-2007/038995
- MOURE F ET AL: "Coupling process for plasma protein fractionation using ethanol precipitation and ion exchange chromatography" MEAT SCIENCE, ELSEVIER SCIENCE, GB, vol. 64, no. 4, August 2003 (2003-08), pages 391-398, XP002373788 ISSN: 0309-1740
- BURNOUF T: "Chromatography in plasma fractionation: benefits and future trends" JOURNAL OF CHROMATOGRAPHY B : BIOMEDICAL APPLICATIONS, ELSEVIER SCIENCE PUBLISHERS, NL, vol. 664, no. 1, 3 February 1995 (1995-02-03), pages 3-15, XP004043701 ISSN: 0378-4347
- BURNOUF T ET AL: "NANOFILTRATION OF PLASMA-DERIVED BIOPHARMACEUTICAL PRODUCTS" HAEMOPHILIA, BLACKWELL SCIENCE, OXFORD, GB, vol. 9, no. 1, January 2003 (2003-01), pages 24-37, XP001181709 ISSN: 1351-8216
- BAKALTCHEVA IRINA ET AL: "Freeze-dried whole plasma: evaluating sucrose, trehalose, sorbitol, mannitol and glycine as stabilizers." THROMBOSIS RESEARCH 2007, vol. 120, no. 1, 2007, pages 105-116, XP022035911 ISSN: 0049-3848
- PIKAL M J ET AL: "THE EFFECTS OF FORMULATION VARIABLES ON THE STABILITY OF FREEZE- DRIED HUMAN GROWTH HORMONE" PHARMACEUTICAL RESEARCH, NEW YORK, NY, US, vol. 8, no. 4, 1 April 1991 (1991-04-01), pages 427-436, XP000577700 ISSN: 0724-8741
- RIPOCHE J ET AL: "ISOLATION OF 2 MOLECULAR POPULATIONS OF HUMAN COMPLEMENT FACTOR H BY HYDROPHOBIC AFFINITY CHROMATOGRAPHY" BIOCHEMICAL JOURNAL, vol. 221, no. 1, 1984, pages 89-96, XP009085096 ISSN: 0264-6021 cited in the application
- H. Brandstätter ET AL: "Purification and biochemical characterization of functional complement factor H from human plasma fractions", Vox Sanguinis, vol. 103, no. 3, 12 October 2012 (2012-10-12), pages 201-212, XP055126784, ISSN: 0042-9007, DOI: 10.1111/j.1423-0410.2012.01610.x
- NAGASAWA, S. AND STROUD, R.M.: 'Purification and characterization of a macromolecular weight cofactor for C3b-inactivator, C4bC3bINA-cofactor, of human plasma' MOLECULAR IMMUNOLOGY vol. 17, pages 1365 - 1372

## Description

### Field of invention

The present invention relates to industrial large-scale production processes for human plasma-derived complement Factor H preparations for therapeutic use.

### Background of the invention

Among experts complement Factor H is discussed to play a central role in certain rare, but severe diseases associated with complement activation via the alternative pathway [Membranoproliferative Glomerulonephritis type II (Dense Deposit Disease): An Update, J Am Soc Nephrol 16: 1392-1404, 2005; Complement and diseases: Defective alternative pathway control results in kidney and eye diseases; P.F. Zipfel et al. / Molecular Immunology 43 (2006) 97-106]. There are examples reported in the literature of treating affected patients successfully with fresh frozen plasma to substitute absent or dysfunctional complement Factor H [Licht et al. American Journal of Kidney diseases, Vol 45, No 2 (February), 2005: pp415-421 / Successful plasma therapy in hemolytic uremic syndrome with complement Factor H deficiency, S. Nathanson, V. Fremeaux-Bacchi, G. Deschenes, Pediatric Nephrology Vol 16 No. 7, (2001) pp 554-556 / Nephrol. Dial. Transplant. 2002, 17: 684]. However, this treatment with fresh frozen plasma is related to certain disadvantages e.g. the large volume necessary to infuse the intended amount of complement Factor H, the risk of allergic reactions associated with the large amount of concomitant infused proteins and the risk of transmitting blood-borne pathogens.

To date no pharmaceutical composition comprising complement Factor H concentrate exists, to allow the substitution of complement Factor H in a more convenient, better tolerable (less volume, less impurities) and safer (lower risk to transmit blood-borne pathogens) way. In addition, a complement Factor H concentrate would allow for higher dosing, which might be of advantage in certain cases. The demand for such a complement Factor H concentrate for therapeutic use is therefore clearly evident.

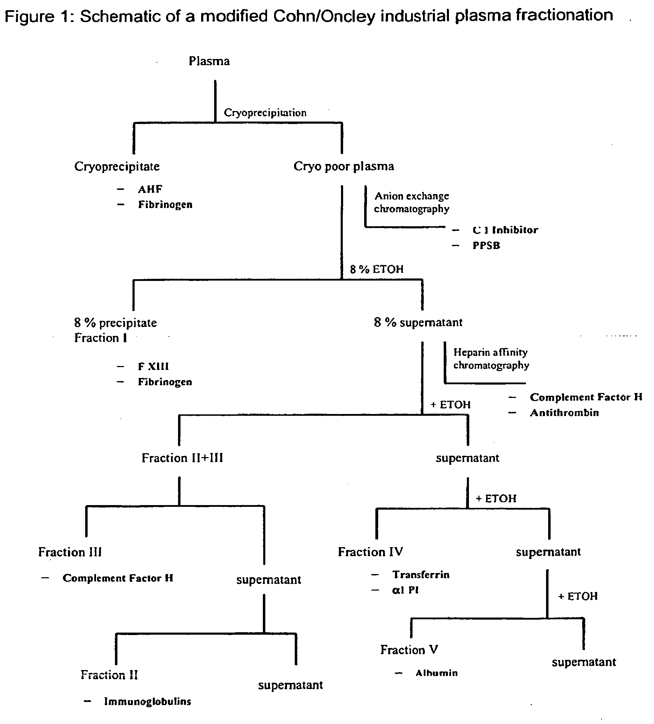

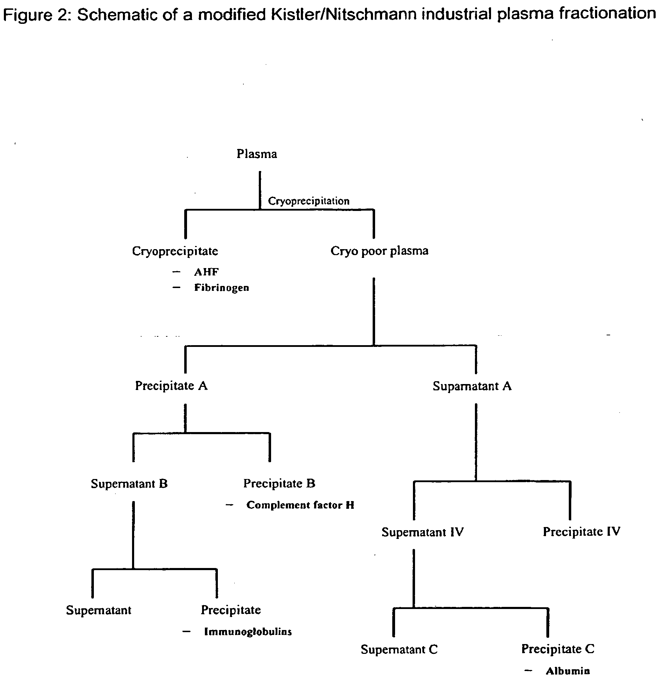

An appropriate source for the manufacturing of a complement Factor H concentrate is human blood plasma in which complement Factor H is found in a concentration of approx. 500 (200-600) mg/L. Human blood plasma is industrially utilized for decades for the manufacturing of widely established and accepted plasma-protein products such as e.g. human albumin, immunoglobulin preparations (IgG), clotting factor concentrates (clotting Factor VIII, clotting Factor IX, prothrombin complex etc.) and inhibitors (Antithrombin, C1-inhibitor etc.). In the course of the development of such plasma-derived drugs, plasma fractionation methods have been established, leading to intermediate products enriched in certain protein fractions, which then serve as the starting material for the according plasma-protein product. Typical processes are reviewed e.g. in Schultze HE, Heremans JF; Molecular Biology of Human Proteins. Volume I: Nature and Metabolism of Extracellular Proteins 1966, Elsevier Publishing Company; p. 236-317 and simplified schematics of such processes are given in figures 1 & 2. These kinds of separation technologies allow for the manufacturing of several therapeutic plasma-protein products from the same plasma donor pool being economically advantageous over producing only one plasma-protein product from one donor pool and have, therefore, being adopted as the industrial standard in blood plasma fractionation.

Modem industrial plasma fractionation processes are subject to the control of regulatory authorities (e.g. FDA, EMEA) and strict regulations (cGMP) have to be met regarding the robustness of processes and the constant quality of the resulting pharmaceutical products. Changes to licensed manufacturing processes are complex in time and effort regarding validation and, in addition, are at risk of adversely affecting the quality of the pharmaceutical product. In the development of industrial scale manufacturing processes for new therapeutic proteins from human plasma as it is the case with complement Factor H, it is thus desirable to keep the integrity of already existing/licensed processes.

The present invention provides methods of extraction of complement Factor H from side fractions of already existing/licensed large/industrial scale plasma fractionation processes and further processing into pharmaceutical preparations at industrial large scale.

Large/industrial scale with regard to the present invention means a production procedure based on at least 200 L plasma, preferentially at least 500 L, even more preferentially at least 2000 L human plasma. Regarding production, the claimed processes starting from human plasma shall be based on the sub fractionation of typical industrial intermediates obtained by above stated plasma fractionation processes. The supernatant of the 8% ethanol-precipitate (method of Cohn et al.; Schultze et al. (see above), p. 251), precipitate II+III (method of Oncley et al.; Schultze et al. (see above) p. 253) or precipitate B (method of Kistler and Nitschmann; Schultze et al. (see above), p. 253) are examples of a source of complement Factor H compatible with industrial scale plasma fractionation in such a way, that complement Factor H is enriched in side fractions not affecting already established and licensed manufacturing processes of plasma products which are under survey of pharmaceutical regulatory authorities.

During manufacturing of antithrombin III (AT) from the 8 % ethanol-supematant by heparin affinity chromatography, complement Factor H, under certain conditions, binds to the immobilized Heparin and can be purified from non AT containing (waste) fractions. In cases where no AT is adsorbed from the 8 % supernatant, or complement Factor H does not bind to the Heparin affinity resin under the conditions given, complement Factor H is enriched in side precipitates of the established immunoglobulin manufacturing processes and is regarded as an impurity.

An additional aspect in the manufacturing of therapeutic plasma protein preparations is the potential risk to transmit blood-born pathogens, e.g. hepatitis B and C virus (HBV and HCV), human immunodeficiency virus (HIV), human T leukaemia virus (HTLV-I), parvovirus B19, hepatitis A virus (HAV), cytomegalovirus (CMV) [reviewed in: Virus safety of human blood, plasma, and derived products; Thrombosis Research, Volume 107, Supplement 1, 31 October 2002, Pages S39-S45 Lutz G. Guertler)] and, even though with a lower risk, causative agents of transmissible spongiform encephalopathies (TSEs).

The present invention provides pharmaceutical complement Factor H preparations produced by the methods of the invention. The so produced preparations have complement Factor H concentrations of 5 mg/mL or higher, preferably 50 mg/mL or higher, more preferably 100 mg/mL or higher.

### Background Art

Complement Factor H was first described by Nilsson & Mueller-Eberhard (J Exp Med, 122, pp 277-298) and several methods for purification are described in the literature (e.g. Alsenz et al, Biochem J (1984), 224, 389-398 / Fearon, J. Immunol. (1977) 119, 1248-1252 / Crossley et al., Biochem J (1980), 191. 173-182). However, these methods are typical small-scale technologies to obtain a single protein of high purity for characterization means and not suitable for routine manufacturing of plasma-protein drugs. Also the purification procedures cited in EP0222611 are typical laboratory methodologies exclusively developed for purification of only complement Factor H from human plasma or serum.

Patent application PCT/EP2006/005631 discloses a process for the purification of functional complement Factor H based on intermediates obtained by large-scale fractional precipitation of human plasma or serum with ethanol, which does not affect the regulatory approval of other therapeutic proteins which are commercially obtained from plasma, as well as a process for the purification of functional complement Factor H in which the purification is based on the supernatant of the 8% ethanol-precipitate according to the method of Cohn or precipitate III according to the method of Oncley or precipitate B according to the method of Kistler and Nitschmann. PCT/EP2006/005631 also discloses a process for the purification of functional complement Factor H, based on intermediates obtained by large-scale fractional precipitation of human plasma or serum with ethanol in combination with chromatographic procedures, wherein these chromatographic procedures may be based on precipitation with polyethylene glycol (Nagasawa S, Stroud RM; Mol Immunol 1980; 17: 1365-72), affinity chromatography via immobilized heparin (citation as before), ion exchange chromatography (Crossley LG, Porter RR; Biochem J 1980; 191 : 173-82) and hydrophobic interaction chromatography (Ripoche J, Al Salihi A, Rousseaux J, Fontaine M ; Biochem J 1984; 221 , 89-96). Finally PCT/EP2006/005631 discloses a process for the purification of functional complement Factor H from an eluate separating complement Factor H from antithrombin III.

Moure et al. (2003) Meat Science Vol. 64, No. 4, pages 391 - 398 describes a coupling process for plasma protein fractionation using ethanol precipitation and ion exchange chromatography.

Bumouf (1995) Journal of Chromatography B 664, pages 3 - 15, is a review on the benefits and future trends of chromatography in plasma fractionation.

Burnouf and Radosevich (2003) Haemophilia 9, pages 24 - 37 is a review article on nanofiltration of plasma-derived biopharmaceutical products.

Bakaltcheva et al. (2007) Thrombosis Research 120, pages 105 - 116 evaluates sucrose, trehalose, sorbitol, mannitol and glycine as stabilizers for freeze-dried whole plasma.

Pikal et al. (1991) Pharmaceutical Research Vol. 8, No. 4, pages 427 - 436 investigates the effects of formulation variables on the stability of freeze-dried human growth hormone.

EP 0 317 376 A1 relates to a process for the preparation of a Factor IX concentrate of high purity.

EP 1 739 093 A1 describes processes for the separation of plasma proteins, in particular of fibrinogen and Factor XIII.

### Summary of the invention

The present invention provides industrial large-scale production processes for human plasma-derived complement Factor H preparations for therapeutic use, utilizing side fractions from already established and licensed fractionation processes not affecting the integrity of said licensed processes combined with methods for virus/pathogen inactivation and/or elimination.

### Detailed description of the invention

The invention therefore relates to a method for purification of complement Factor H in a solution comprising the steps of:
a) providing a fraction comprising complement Factor H by large-scale fractional precipitation of human plasma or serum with ethanol, wherein at least one further commercially obtainable pharmaceutical protein is produced by said large-scale fractional precipitation process
b) further purifying complement Factor H by at least one purification method selected from the group:
   I. Heparin affinity chromatography
   II. Hydrophobic interaction chromatography (HIC)
   III. Anion exchange chromatography (AEC)
   IV. Cation exchange chromatography (CEC)
   V. Hydroxyapatite chromatography (HAC)
   VI. Immunoaffinity chromatography
c) treating the complement Factor H containing fraction at least once before, during or after step b) of said purification process with a virus/pathogen inactivation and/or elimination method;
wherein the purification of complement Factor H comprises the following process steps:
I. an initial anion exchange chromatography
II. followed by a hydrophobic interaction chromatography
III. followed by pasteurization
IV. followed by a heparin affinity chromatography
V. followed by nanofiltration.

The further pharmaceutical protein could by way of nonlimiting example be one or more of the proteins selected from albumin, antithrombin III, Factor VIII, Factor IX, fibrinogen, Factor XIII, thrombin (FIIa), FVII/IIa, prothrombin-complex preparations (PPSB), alpha-1 proteinase inhibitor, C1 Inhibitor, immunoglobulins, transferrin, butyrylcholinesterase.

The virus/pathogen inactivation or elimination technology may by way of nonlimiting example be selected from pasteurization, solvent/detergent treatment or nanofiltration.

Pasteurization in the sense of the invention relates to methods exposing a liquid Factor H composition to a temperature of 60 ± 1 °C for a time of at least 10 h.

Solvent/detergent treatment in the sense of the invention relates to methods of treating a liquid Factor H composition with formulations of tri-n-butylphosphate and a detergent selected form the group of Triton X-100, Tween or sodium cholate.

Nanofiltration in the sense of the invention relates to methods for removing viruses and/or causative agents of transmissible spongiform encephalopathies (TSEs) from a liquid Factor H composition, said method comprising filtering said Factor H solution through a filter having a pore size of ≤ 80 nm.

The different purification technologies may be repeated one or several times or combined with each other depending on the desired level of purity for the final pharmaceutical grade complement Factor H.

Chromatography in the sense of the invention relates to either one of the following techniques:
- applying complement Factor H in solution to a respective binding matrix, binding complement Factor H to said matrix, optionally washing said matrix one or more times, under condition where complement Factor H remains bound to the matrix, then applying a solution to the matrix with bound complement Factor H which causes the elution of complement Factor H from the binding matrix, or
- applying complement Factor H in solution to a respective binding matrix, binding impurities to said matrix whilst leaving the Factor H in the unbound fraction

Optionally said processes will also comprise a delipidation/precipitation step, or the addition of a protease inhibitor or the removal of a protease.

The invention extends to complement Factor H preparations obtained by any of the above mentioned processes.

Pharmaceutical preparation in the sense of the invention is any composition comprising complement Factor H which can be used in medicine.

A preferred embodiment of the above discussed methods for purification of Factor H is starting the purification from a fraction comprising complement Factor H obtained by large-scale fractional precipitation of human plasma or serum with ethanol which is a waste fraction, e.g. a fraction from which presently no pharmaceutical product is obtained.

### Figures:

- Figure 1 :: Schematic of a modified Cohn/Oncley industrial plasma fractionation
- Figure 2:: Schematic of a modified Kistler/Nitschmann industrial plasma fractionation

### Examples:

The complement Factor H concentrations referenced in the following sections are obtained by a commercially available radial immunodiffusion (RID) assay ("HUMAN FACTOR H 'NL' BlNDARID™"; Product Code: RN030.3; The Binding Site Limited, Birmingham, UK)

### 1. Providing a fraction comprising complement Factor H

### a. 8 % ethanol (Fraction I) supernatant in Cohn(Oncley) fractionation

When Cohn/Oncley fractionation methods are applied, complement Factor H is comprised in the supernatant of the 8% ethanol precipitation. In the case of separating antithrombin III (AT) from the 8 % ethanol-supernatant by Heparin affinity chromatography, complement Factor H, under certain conditions, binds to the immobilized Heparin and can be purified from non AT containing (waste) fractions (see below).

### b. Fraction III in Cohn(Oncley) fractionation

If complement Factor H is not recovered from the 8% ethanol supernatant, it is enriched in Fraction III, a side fraction of typical immunoglobulin manufacturing procedures.

### c. Precipitate B in Kistler/Nitschmann fractionation

When Kistler/Nitschmann fractionation methods are applied, complement Factor H is enriched in Precipitate B, a side fraction of typical immunoglobulin manufacturing procedures.

### 2. Further purification methods

### a. Heparin affinity chromatography

As complement Factor H has a known affinity to glycosaminoglycans, heparin affinity chromatography is a suitable purification method. heparin or heparin derivatives or heparin mimetics immobilized to agarose-based or polymeric base-matrices can be used. Alternative heparin-like affinity-ligands of comparable function are e.g. chondroitin sulfate, heparan sulfate, dermatan sulfate, keratan sulfate, or synthetic oligomers, e.g. cellubiose sulfate ester (Matrex ™ Cellufine Sulfate), or therapeutic heparin analogues e.g. sulfated oligosaccharides representing partial heparin structures (e.g. sulfated pentasaccharides, sulfated tetrasaccharides).

The source fraction (complement Factor H rich wash from heparin affinity chromatography out of 8 % supernatant from a Cohn/Oncley fractionation, or redissolved Fraction III from a Cohn/Oncley fractionation, or redissolved Precipitate B from a Kistler/Nitschmann fractionation or such fraction delipidated by e.g. the method described under 2.g.) are formulated to a corresponding conductivity of < 25 mS/cm (25 °C) and a pH of 5 - 9 in suitable buffer systems (e.g. saline or phosphate-buffer or citrate buffer). The so conditioned solution is then applied to a Heparin-Affinity-Resin column (heparin immobilized on Toyopearl HW-65). The column is washed with low salt buffer solution (25 mmol/L tri-sodium citrate) and subsequently the complement Factor H is eluted by an ascending NaCl gradient (0-1 mol/L) in the same buffer.

The 8 % supernatant of a modified Cohn/Oncley fractionation (see Figure 1) was subjected to a heparin-affinity chromatography. Approx. 0.08 L Heparin-Affinity-Resin (heparin immobilized on Toyopearl HW-65) were added per liter of 8 % supernatant and the suspension was stirred for approx. 1 h in the cold. Subsequently, the resin was recovered by filtration and transferred into a chromatography column. Complement Factor H was eluted from the resin utilizing a solution containing approx. 0.25 mol/L tri-sodium citrate dihydrate at pH 6.25 whilst ATIII remained bound to the column.

**Table 1: Complement Factor H purified by heparin-affinity chromatography based on 8% ethanol (Fraction I) supernatant of Cohn(Oncley) fractionation**

| Sample | Volume [L] | Absorption 280 nm | Factor H [mg/L] | Factor H [%] |
|---|---|---|---|---|
| 8 % supernatant | 2955 | n.a. | 308 | 100 |
| Citrate eluate | 500 | 8.6 | 1530 | 84 |

This process for purifying complement Factor H does not affect the licensed manufacturing processes of the ATIII-product or the products made from fractionation of the heparin-affinity supernatant (e.g. IgG, albumin, α-1 PI, etc.).

### b. Hydrophobic Interaction Chromatography (HIC)

Complement Factor H, under certain conditions, binds to suitable HIC resins and can be desorbed by buffer systems of low ionic strength. Suitable HI ligands are e.g. phenyl, octyl, butyl, methyl as well as modem multi-modal ligands (e.g. hexylamine, phenylpropylamine, 4-mercapto-ethyl-pyridine) immobilized to agarose-based or polymeric base-matrices. Examples for commercially available HIC-media or multimodal-media are e.g. phenyl sepharose, octyl sepharose, butyl sepharose, Capto™ MMC (all GE Healthcare), Toyopearl Phenyl-650, Toyopearl Butyl-650. Toyopearl Hexyl-650 (all Tosoh Biosciences). Macro-Prep Methyl HIC Support, Macro-Prep t-Butyl HIC Support (Bio-Rad), HEA HyperCel; PPA HyperCel, MEP HyperCel (all Pall). In a preferred embodiment, butyl is used as the ligand resulting in the best complement Factor H recovery.

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation or such fraction delipidated by e.g. the method described under 2.g.) are formulated to a corresponding conductivity of > 100 mS/cm (25 °C) and a pH of 5 - 9 in suitable buffer systems (e.g. saline or phosphate-buffer or citrate buffer). The so conditioned solution is then applied to a column packed with Butyl Sepharose FF (GE Healtheare). The column is washed with high salt buffer solution (25 mmol/L tri-sodium citrate, 2 mol/L NaCl) and subsequently the complement Factor H is eluted by an descending NaCl gradient ( 2 - 0 mol/L) in the same buffer.

The complement Factor H rich wash from Heparin Affinity chromatography out of 8 % supernatant was delipidated (see below) and adjusted to a conductivity of approx. 130 mS/cm by addition of NaCl and a pH value of 5.5. The solution was applied to a column packed with Butyl Sepharose FF (GE Healthcare) and the complement Factor H was bound to the resin under said conditions. After washing the column with high salt buffer solution (2 mol/L NaCl, 200 mmol/L tri-sodium citrate dihydrate pH 5.5) the complement Factor H was eluted by a step gradient to 30 % of the initial NaCl concentration in the same buffer.

**Table 2: Complement Factor H purified by hydrophobic interaction chromatography based on 8% ethanol (Fraction I) supernatant of Cohn(Oncley) fractionation**

| Sample | Absorption 280 nm | Factor H [mg/L] | Factor H [%] |
|---|---|---|---|
| PEG filtrate | 4.1 | 915 | 100 |
| Butyl Sepharose eluate | 1.5 | 1503 | 75 |

### c. Anion exchange chromatography AEC)

Complement Factor H, under certain conditions, binds to suitable AEC resins and can be desorbed by buffer systems of high ionic strength. Suitable AEC ligands are e.g. diethylaminoethyl (DEAE), quaternary aminoethyl (QAE) or quaternary ammonium (Q) immobilized to agarose-based or polymeric base-matrices. Commercially available anion-exchange-media are e.g. DEAE-Sepharose, Q-Sepharose (GE Healthcare), Toyopearl DEAE-650, Toyopearl Super Q-650 (Tosoh Biosciences), Fractogel EMD DEAE, Fractogel EMD TMAE (Merck KGaA), Macro-Prep DEAE Support, Macro-Prep High Q Support (BioRad), DEAE HyperD, Q HyperD, DEAE Trisacryl, DEAE Sperodex (all Pall). In a preferred embodiment, quaternary ammonium immobilized to a base-matrix at least partially consisting of dextran is used (Q-Sepharose XL, Capto ™ Q) due to significant higher complement Factor H binding capacity compared to other AEC media.

Those AEC media having a low(er) binding capacity for Factor H can be used in negative mode, which means that, under certain conditions only impurities bind to the matrix whilst Factor H remains in the unbound fraction. Examples for AEC media with low(er) binding capacity fro Factor H are e.g. DEAE Sepharose FF, Toyopearl DEAE-650, MacroPrep DEAE Support.

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation or such fraction delipidated by e.g. the method described under 2.g.) is formulated to a corresponding conductivity of < 6 mS/cm (25 °C) and a pH of 7 - 9 in suitable buffer systems (e.g. saline or phosphate-buffer or citrate buffer). The so conditioned solution is then applied to a column packed with Q-Sepharose XL ( GE Healthcare ). The column is washed with low salt buffer solution (25 mmol/L tri-sodium citrate, 2 mol/L NaCl) and subsequently the complement Factor H is eluted by an ascending NaCl gradient (0 - 1 mol/L) in the same buffer.

The complement Factor H rich side fraction from the Heparin-affinity step according to example 1 or, alternatively, the delipidated material according to 2 g was adjusted to a pH value of 9 and diluted to a conductivity <6 mS/cm. The diluted solution was applied to a column packed with Capto ™ Q (GE Healthcare) and the complement Factor H was bound to the resin under said conditions. After washing the column with low salt buffer solution (25 mmol/L TRIS pH 7.5 ) the complement Factor H was eluted by an ascending NaCl gradient (0-0.5 mol/L) in the same buffer.

**Table 3: Complement Factor H purified by anionic exchange chromatography based on 8% ethanol (Fraction I) supernatant of Cohn(Oncley) fractionation**

| Sample | Absorption 280 nm | Factor H [mg/L] | Factor H [%] |
|---|---|---|---|
| PEG filtrate | 3.6 | 735 | 100 |
| Capto Q eluate | 2.9 | 2355 | 76 |

The source fraction (complement Factor H rich wash from heparin Affinity chromatography out of 8 % supernatant from a Cohn/Oncley fractionation, or redissolved Fraction III from a Cohn/Oncley fractionation, or redissolved Precipitate **B** from a Kistler/Nitschmann fractionation or such fraction delipidated by e.g. the method described under 2.g.) is formulated to a corresponding conductivity of < 20 mS/cm (25 °C) and a pH of 5 - 9 in suitable buffer systems (e.g. saline or phosphate-buffer or citrate buffer). The so conditioned solution is then applied to a column packed with DEAE-Sepharose FF ( GE Healthcare ). The Factor H, under these conditions is comprised in the unbound fraction. Optionally, the column is post-washed with low salt buffer solution to recover trace amounts of Factor H left in the liquid phase of the column package.

### d. Cation exchange chromatography (CEC)

Complement Factor H, under certain conditions, binds to suitable CEC resins and can be desorbed by buffer systems of high ionic strength. Suitable CEC ligands are e.g. carboxymethyl (CM), sulfopropyl (SP) or methyl sulfonate (S) or similar, immobilized to agarose-based or polymeric base-matrices. Commercially available cation-exchange-media are e.g. CM-Sepharose, SP-Sepharose (GE Healthcare), Toyopearl CM-650, Toyopearl SP-650 (Tosoh Biosciences), Fractogel EMD SO₃⁻, Fractogel EMD COO⁻ (Merck KGaA), Macro-Prep CM Support, Macro-Prep High S Support (BioRad), CM HyperD, S HyperD, CM Trisacryl, SP Trisacryl, SP Sperodex (all Pall).

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation or such fraction delipidated by e.g. the method described under 2.g.) is formulated to a corresponding conductivity of < 20 mS/cm (25 °C) and a pH of 5 - 7 in suitable buffer systems (e.g. saline or phosphate-buffer or citrate buffer). The so conditioned solution is then applied to a column packed with Fractogel EMD SO₃⁻ (Merck). The column is washed with low salt buffer solution (25 mmol/L tri-sodium citrate) and subsequently the complement Factor H is eluted by an ascending NaCl gradient (0 - 1 mol/L) in the same buffer.

The complement Factor H rich side fraction from the Heparin-affinity step according to example 1 or, alternatively, the delipidated material according to section 2 g was adjusted to a pH value of 6 and diluted to a conductivity < 6 mS/cm. The diluted solution was applied to a column packed with SP Sepharose FF (GE Healthcare) and the complement Factor H was bound to the resin under said conditions. After washing the column with low salt buffer solution (25 mmol/L tri-sodium citrate dihydrate pH 6.5 ) the complement Factor H was eluted by an ascending NaCl gradient (0 - 0.5 mol/L) in the same buffer.

**Table 4: Complement Factor H purified by cationic exchange chromatography based on 8% ethanol (Fraction I) supernatant of Cohn(Oncley) fractionation**

| Sample | Absorption 280 nm | Factor H [mg/L] | Factor H [%] |
|---|---|---|---|
| PEG filtrate | 3.9 | 840 | 100 |
| SP Sepharose eluate | 5.6 | 3500 | 95 |

### e. Hydroxyapatite chromatography (HAC)

Complement Factor H, can be purified utilizing composits of calciumphosphate, fluoroapatite, hydroxyapatite or hydroxyapatite-like preparations as solid phase for chromatography. Such media can be e.g. Bio-Gel Hydroxyapatite HT an HTP (Bio-Rad), Ceramic Fluoroapatite, Ceramic Hydroxyapatite (Bio-Rad), HA Ultrogel (Pall) and the like.
Complement Factor H, under certain conditions, bind to the HA-matrix and can be desorbed by buffer systems containing rising concentrations of salt, preferably phosphate
The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation or such fraction delipidated by e.g. the method described under 2.g.) is formulated to a corresponding conductivity of < 15 mS/cm (25 °C) and a pH of 5 - 9 in suitable buffer systems (e.g. saline or phosphate-buffer or citrate buffer). The so conditioned solution is then applied to a column packed with HA Ultrogel (Pall). The column is washed with low salt buffer solution (e.g. 10 mmol/L potassium phosphate) and subsequently the complement Factor H is eluted by an ascending phosphate gradient (0 -1 mol/L).

### f. Immunoaffinity chromatography

Complement Factor H can be captured and purified by immunoaffinity chromatography. Complement Factor H specific antibodies are immobilized on suitable chromatography matrices. Suitable matrices for large scale applications are pre-activated resins e.g. epoxy activated Sepharose 6B, EAH Sepharose 4B. Amino Sepharose 6 FF, 6-AKS Sepharose 4FF, Toyopearl AF Epoxy, Toyopearl AF Amino, Toyopearl AF Tresyl or the like. Coupling procedures are known by persons trained in the art. The complement Factor H containing solutions are applied onto such immunoadsorbents at conditions where complement Factor H binds to the immobilized antibodies. Unbound impurities are washed out using suitable buffer systems (e.g. citrate buffer, phosphate buffer, HEPES buffer) and complement Factor H is subsequently eluted from the immunoadsorbent utilizing e.g. high enough concentrations of chaotropic salts (e.g. thiocyanate, lithium bromide etc.) or low pH buffer systems (e.g. glycine pH 2-3) or high pH buffer systems (e.g.TRIS buffer pH 9).

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation or such fraction delipidated by e.g. the method described under 2.g.) is formulated in HEPES at pH 7.6. The so conditioned solution is then applied to a column packed with an immunoadsorbent carrying anti-Factor H antibodies. The column is washed with the said HEPES buffer and subsequently the complement Factor H is eluted by an ascending KCNS gradient (0 -1 mol/L).

### 3. Optional further purification steps

### a) Detipidation

As the complement Factor H rich fractions referenced under "1. Providing a fraction comprising complement Factor H" may contain lipid or lipoprotein impurities, it might be necessary to remove these prior to subsequent chromatography based purification techniques to protect the chromatography resins from cumulative accumulation of said lipids ("column fouling"). Lipid impurities can be removed from protein solutions by means of e.g. precipitation or adsorption. Suitable precipitants are e.g. polyethylene glycols (PEG) or ammonium sulfate, suitable adsorbents are e.g. silica powders ("fumed silica"; Aerosil 200, Aerosil 380), dextran sulfates or fluorocarbons (Freon-113).

The source fraction (complement Factor H rich wash from heparin affinity chromatography out of 8 % supernatant from a Cohn/Oncley fractionation, or redissolved Fraction III from a Cohn/Oncley fractionation, or redissolved Precipitate B from a Kistler/Nitschmann fractionation) is formulated to a corresponding conductivity of < 40 mS/cm (25 °C) and a pH of 5 - 9 in suitable buffer systems (e.g. saline or phosphate-buffer or citrate buffer) at a temperature of 0 - 40 °C. To the such conditioned complement Factor H containing solution, PEG 4000 is added to result in a final concentration of 2 - 10 % w/v. Alternatively, Polyethylene glycols of other molecular weights can be used where higher molecular weights require lower final concentrations and vice versa. After an incubation time sufficient to achieve precipitation of the targeted impurities, the precipitate is removed by filtration or centrifugation.

The complement Factor H rich wash (32 mS/cm, pH 6.2) from Heparin Affinity chromatography out of 8 % supernatant was warmed up to 30 °C. A PEG 4000 concentrate (25 % w/v) was added to achieve a final PEG concentration of 7.5 % w/v. The mixture was stirred for 30 min. (30 °C) and filtered through a combination of depth filters following addition of 10 g/L perlite filter aid.

**Table 5: Deplipidation of complement Factor H rich wash from heparin affinity chromatography of 8 % supernatant from a Cohn/Oncley fractionation using PEG precipitation**

| Sample | Abs. 280 nm | Factor H | | Triglyceride | | Cholesterol | |
|---|---|---|---|---|---|---|---|
| | | [mg/L] | [%] | [mg/dl] | [%] | [mg/dl] | [%] |
| Citrate eluate | 8.4 | 1596 | 100 | 75 | 100 | 162 | 100 |
| PEG 4000 filtrate | 3.6 | 915 | 78.3 | 3 | 5.5 | 17 | 14.3 |

Fraction III was resuspended in citrate buffer solution to a conductivity of 30.5 mS/cm and a pH value of 6.3 (25 °C). Polyethyleneglycol 4000 was added to a final concentration of 7.5 %. Subsequently the suspension was filtered through a combination of depth filters.

**Table 6: Deplipidation of complement Factor H rich citrate buffer resuspension of Fraction III from a Cohn/Oncley fractionation using PEG precipitation**

| Sample | Absorption 280 nm | Factor H [mg/L] | Factor H [%] |
|---|---|---|---|
| Fraction III suspension | 9.6 | 318 | 100 |
| Filtrate | 4.3 | 178 | 59 |

Precipitate B was resuspended in citrate buffer solution to a conductivity of 29.5 mS/cm and a pH value of 6.3 (25 °C). Polyethylene glycol 4000 was added to a final concentration of 7.5 %. Subsequently the suspension was filtered through a combination of depth filters.

**Table 7: Deplipidation of complement Factor H rich citrate buffer resuspension of Precipitate B from a Kistler/Nitschmann fractionation using PEG precipitation**

| Sample | Absorption 280 nm | Factor H [mg/L] |
|---|---|---|
| Filtrate | 4.8 | 268 |

### b. Removal or inhibition of proteolytic impurities

As the complement Factor H rich side fractions referenced under "1. Providing a fraction comprising complement Factor H" may contain proteolytic impurities, able to cleave the complement Factor H molecule leading to truncated forms of impaired function, it might be necessary to implement suitable methods to remove or inhibit these impurities from/in the complement Factor H containing solutions as early as possible in the manufacturing process.
Suitable measures to remove such proteolytic impurities are chromatographic methods utilizing protease inhibitors immobilized to agarose-based or polymeric base-matrices. Such protease inhibitors can be e.g. benzamidine, 4-aminobenzamidine, benzamidine hydrochloride and derivatives thereof, lysine and derivatives thereof, 6-aminohexanoic acid (ε-aminocaproic acid) and derivatives thereof, trans-4-(Aminomethyl)-cyclohexanecarboxylic acid (tranexamic acid) and derivatives thereof, 4-(2-Aminoethyl)benzenesulfonylfluoride (AEBSF) and derivatives thereof, (4-Amidino-Phenyl)-Methane-Sulfonyl Fluoride (APMSF) and derivatives thereof, 3,4-dichloroisocoumarin (DCI) and derivatives thereof, Acetyl-leucyl-leucyl-arginal (Leupeptine) and derivatives thereof, aprotinin and derivatives thereof, soybean trypsin inhibitor and derivatives thereof, α2-antiplasmin and derivatives thereof, antithrombin (antithrombin III) and derivatives thereof. Commercially available chromatography media are e.g. ECH Lysine Sepharose 4 B, Benzamidine Sepharose 6B (GE Healthcare), p-Aminobenzamidine Agarose 6XL (Prometic) or the like.
Alternatively to their removal, proteolytic impurities can be inhibited in the complement Factor H containing solutions by adding one ore more protease inhibitor out of the above mentioned group to the solution and, subsequently, remove the enzyme/inhibitor complex by means of the following purification steps. Another approach to remove proteolytic impurities is the use of immobilize dyes like e.g. Cibacron Blue F3GA or derivatives thereof, other triazine dyes like Procion Red HE-3B and derivatives thereof, or Procion Green H-4G and derivatives thereof, Reactive Red 120 and derivatives thereof, Reactive Green 19 and derivatives thereof, Reactive Yellow 86 and derivatives thereof, Reactive Orange 14 and derivatives thereof and the like, immobilized to agarose-based or polymeric base matrices.

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation) is formulated to a corresponding conductivity of < 40 mS/cm (25 °C) and a pH of 5 - 9 in suitable buffer systems (e.g. saline or phosphate-buffer or citrate buffer) at a temperature of 0 - 40 °C. The solution is passed through a column packed with e.g. ECH Lysine Sepharose 4B (GE Healthcare) and the column is washed subsequently with 3 column volumes of buffer solution. Alternatively the lysine Sepharose can be incubated batch-wise (added to the solution and incubated while agitating) and removed by filtration.

Benzamidine or benzamidine*HCl or 4-aminobenzamidine or 4-aminobenzamidine*2HCl is added to a complement Factor H containing solution to a concentration of S 200 mmol/L. Subsequently, the solution is subjected to one of the methods or combinations of the methods described under 2 a-g. 3 a-f. 4 a-c. 5 a-f.

### 4. Combination of purification methods

In order to obtain a complement Factor H product of higher purity than what is achieved by the aforesaid methods, each of the methods 2 a-f and 3 a-b can either be applied a second time or combined with further chromatographic purification steps.

### a. Heparin affinity chromatography,

A complement Factor H containing solution resulting from one of the methods under 2 a-f can be further purified by Heparin affinity chromatography. Suitable ligands and/or media are referenced under 2 a.

Complement Factor H containing solutions are formulated to a corresponding conductivity of < 25 mS/cm (25 °C) and a pH of 5 - 9 in suitable buffer systems (e.g. saline or phosphate-buffer or citrate buffer). The so conditioned solution is then applied to a Heparin-Affinity-Resin column (heparin immobilized on Toyopearl HW-65 ). The column is washed with low salt buffer solution (25 mmol/L tri-sodium citrate) and subsequently the complement Factor H is eluted by an ascending NaCl gradient (0-1 mol/L) in the same buffer.

The complement Factor H rich eluate from cation exchange chromatography on SP Sepharose FF (according to section 2d) was diluted to a conductivity of approx. 6 mS/cm by addition of water and adjusted to a pH value of 5.5. The solution was applied to a Heparin-Affinity-Resin column (Heparin immobilized on Toyopearl HW-65 ). The column is washed with low salt buffer solution (25 mmol/L tri-sodium citrate) and subsequently the complement Factor H is eluted by an ascending NaCl gradient (0 - 1 mol/L) in the same buffer.

**Table 8: Complement Factor H purified by cation exchange chromatography and subsequently by heparin-affinity chromatography based on 8% ethanol (Fraction I) supernatant of Cohn(Oncley) fractionation**

| Sample | Absorption 280 nm | Factor H [mg/L] | Factor H [%] |
|---|---|---|---|
| SP eluate | 5.6 | 3500 | 100 |
| Heparin affinity eluate | 5.2 | 5400 | 83 |

### b. Hydrophobic Interaction Chromatography (HIC)

A complement Factor H containing solution resulting from one of the methods under 2 a-f can be further purified by hydrophobic interaction chromatography. Suitable ligands and/or media are referenced under 2 b.

Complement Factor H containing solutions are formulated to a corresponding conductivity of > 100 mS/cm (25 °C) and a pH of 5 - 9 in suitable buffer systems (e.g. saline or phosphate-buffer or citrate buffer). The so conditioned solution is then applied to a column packed with Butyl Sepharose FF (GE Healthcare ). The column is washed with high salt buffer solution (25 mmol/L tri-sodium citrate, 2 mol/L NaCl) and subsequently the complement Factor H is eluted by an descending NaCl gradient (2 - 0 mol/L) in the same buffer.

The complement Factor H rich eluate from cation exchange chromatography on Fractogel EMD SO₃⁻ (according to section 2d) was adjusted to a conductivity of approx. 130 mS/cm by addition of NaCl and a pH value of 5.5. The solution was applied to a column packed with Butyl Sepharose FF (GE Healthcare) and the complement Factor H was bound to the resin under said conditions. After washing the column with high salt buffer solution (2 mol/L NaCl, 200 mmol/L tri-sodium citrate dihydrate pH 5.5) the complement Factor H was eluted by a step gradient to 30 % of the initial NaCl concentration in the same buffer.

**Table 9: Complement Factor H purified by cation exchange chromatography and subsequently by hydrophobic interaction chromatography based on 8% ethanol (Fraction I) supernatant of Cohn(Oncley) fractionation**

| Sample | Absorption 280 nm | factor H [mg/L] | Factor H [%] |
|---|---|---|---|
| EMDSO₃⁻ eluate | 4.8 | 3800 | 100 |
| Butyl Sepharose eluate | 1.2 | 1700 | 76 |

### c. Anion exchange chromatography (AEC)

A complement Factor H containing solution resulting from one of the methods under 2 a-f can be further purified by anion exchange chromatography. Suitable ligands and/or media are referenced under 2 c.

Complement Factor H containing solutions are formulated to a corresponding conductivity of < 10 mS/cm (25 °C) and a pH of 6-9 in suitable buffer systems (e.g. saline or phosphate-buffer or citrate buffer). The so conditioned solution is then applied to a column packed with Q-Sepharose XL (GE Healthcare). The column is washed with low salt buffer solution (25 mmol/L tri-sodium citrate) and subsequently the complement Factor H is eluted by an ascending NaCl gradient (0 - 1 mol/L) in the same buffer.

### d. Cation exchange chromatography (CEC)

A complement Factor H containing solution resulting from one of the methods under 2 a-f can be further purified by cation exchange chromatography. Suitable ligands and/or media are referenced under 2 d.

Complement Factor H containing solutions are formulated to a corresponding conductivity of < 10 mS/cm (25 °C) and a pH of 5 - 8 in suitable buffer systems (e.g. saline or phosphate-buffer or citrate buffer). The so conditioned solution is then applied to a column packed with SP-Sepharose FF (GE Healthcare ). The column is washed with low salt buffer solution (25 mmol/L tri-sodium citrate) and subsequently the complement Factor H is eluted by an ascending NaCl gradient (0 - 1 mol/L) in the same buffer.

### e. Hydroxyapatite chromatography (HAC)

A complement Factor H containing solution resulting from one of the methods under 2 a-f can be further purified by hydroxyapatite chromatography. Suitable ligands and/or media are referenced under 2 e.

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation) is formulated to a corresponding conductivity of < 15 mS/cm (25 °C) and a pH of 5 - 8 in suitable buffer systems (e.g. saline or phosphate-buffer or citrate buffer). The so conditioned solution is then applied to a column packed with HA Ultrogel (Pall ). The column is washed with low salt buffer solution (e.g. 10 mmol/L potassium phosphate) and subsequently the complement Factor H is eluted by an ascending phosphate gradient (0 - 1 mol/L).

### f. Immunoaffinity chromatography

A complement Factor H containing solution resulting from one of the methods under 2 a-f can be further purified by immunoaffinity chromatography. Suitable ligands and/or media are referenced under 2 f.

### 5. Virus inactivation / elimination technologies

Preferred embodiments of the invention to achieve virus inactivation / elimination are combinations of the following widely accepted methods:
a. Pasteurization (thermal virus inactivation for 10 h at 60 °C in the liquid state)
b. Solvent/Detergent treatment
c. Nanofiltration

### a. Pasteurization

Pasteurization at 60 °C for 10 h is a widely accepted method for virus inactivation of plasma derivatives and has been proven to be effective aginst a broad spectrum of viruses (Sofer G, Virus Inactivation in the 1990s- and into the 21^{st} Century, Biopharm International, June 2003 Supplement).
Pasteurization can be applied to any of the partially or highly purified complement Factor H preparation obtained from any of the methods described under section 1, 2, 3 or 5.

Complement Factor H containing solutions are formulated in suitable buffer solutions (e.g. citrate buffer, phosphate buffer, Tris(hydroxymethyl) aminomethan, HEPES, MOPS etc.) containing NaCl in concentrations up to 1 mol/L or without NaCl at pH values of 5-9. One of the preferred embodiments is 50 mmol/L tri-sodium citrate dihydrate pH 5.5 + 0.9 % NaCl at a protein concentration of Abs. 280 nm = 15. Per L of solution up to 1.5 kg of sucrose are added and dissolved. Other stabilizers might be added, e.g. amino acids like glycine, lysine in concentrations of up to 1 mol/L or even higher. The so stabilized solution is then heated to 60 °C and held at that temperature for 10 h.

HIC-purified complement Factor H (according to section 2 b) was formulated in 0.9 % NaCl, 50 mmol/L tri-sodium citrate dihydrate pH 5.5 at a protein concentration of Abs. 280 nm = 15. Per L of solution 1 kg of sucrose was added and dissolved. The pH value was adjusted to 5.5. Subsequently the stabilized solution was heated to 60 °C and held at that temperature for 10 h. Accordingly the solution was cooled to ambient temperature and diluted 1:3 with citrate buffer pH 5.5.

**Table 10: Pasteurization of a HIC-purified complement Factor H**

| Sample | Absorption 280 nm | Factor H [mg/L] | Factor H [%] |
|---|---|---|---|
| Pre pasteurization | 9.5 | 10550 | 100 |
| Post pasteurization | 9.6 | 10000 | 95 |

### b. Solvent/Detergent (S/D) treatment

Besides Pasteurization, another approved method for virus inactivation is the treatment with combinations of a solvent, e.g. tri-n-butyl phosphate (TNB) and a detergent, e.g. Triton-X100, Polysorbate 80 (Tween 80) or sodium cholate in the liquid state.
S/D treatment can be applied to any of the partially or highly purified complement Factor H preparations obtained from any of the methods described under section 1, 2, 3 or 5, but a suitable method to remove the S/D reagents has to be executed subsequently.

Triton-X100 and tri-n-butyl phosphate are added to a solution containing complement Factor H to a final concentration of 1 % w/v each. The solution is stirred for 4-6 h at 25 - 30 °C. Subsequently, the solvent and detergent are removed by chromatography either in the way that the complement Factor H is bound to the stationary phase and the solvent and detergent are washed out in the unbound fraction or in the way that the solvent and detergent are bound to a suitable ligand (e.g. C-18) leaving the complement factor H in the unbound fraction.

### c. Nanofiltration

Virus filtration is the preferred embodiment to comply with the regulatory guidelines to implement complementary (different modes of action) virus reduction principles/mechanisms. The combination of one of the abovementioned virus inactivation methods (4a or 4 b) with filtration as a virus elimination/reduction method complies perfectly with said regulatory requirements. Suitable virus retentive filters are e.g. Planova ™ 35 nm,
Planova ™ 20 nm (Asahi corporation), Ultipor DV 50 or DV 20 (Pall corporation), Virosart CPV (Sartorius), Viresolve NFR or NFP (Millipore).
Nanofiltration can be applied to any of the partially or highly purified complement Factor H preparations obtained from any of the methods described under section 1, 2, 3 or 5 where the capacity of the used filter may vary with the grade of purity of the solution to be filtered.

Complement Factor H solution was filtered through an accepted virus retentive filter, Planova ™ 20 nm (Asahi corporation). Approx 45 mL Purified complement Factor H solution was filtered through a 0.001 m2 Planova 20 module following prefiltration through a 12.5 cm2 Durapore 0.1 µm filter (Millipore).

**Table 11: Filtration of purified complement Factor H through a Planova™ 20 nm module**

| Sample | Absorption 280 nm | Factor H [mg/L] | Factor H [%] |
|---|---|---|---|
| Before 0.1 µm Filtration | 4.7 | 5860 | 100 |
| Planova 20 nm Filtrate | 4.3 | 4220 | 72 |

### 6. Additional combinations of purification methods

In order to obtain a complement Factor H product of very high purity, each of the aforesaid methods and combinations thereof can either be reenacted or combined with further chromatographic purification steps.

### a. Heparin affinity chromatography

A complement Factor H containing solution resulting from one of the methods or repetitions of the methods or combinations of the methods described under 1-4 can be further purified by Heparin affinity chromatography. Suitable ligands and/or media are referenced under 2 a.

Complement Factor H containing solutions are formulated to a corresponding conductivity of < 25 mS/cm (25 °C) and a pH of 5-9 in suitable buffer systems (e.g. saline or phosphate-buffer or citrate buffer). The so conditioned solution is then applied to a Heparin-Affinity-Resin column (heparin immobilized on Toyopearl HW-65). The column is washed with low salt buffer solution (25 mmol/L tri-sodium citrate) and subsequently the complement Factor H is eluted by an ascending NaCl gradient (0-1 mol/L) in the same buffer.

The complement Factor H rich eluate from a combination of anion exchange chromatography on Q-Sepharose XL, hydrophobic interaction chromatography on Butyl Sepharose FF and pasteurization was formulated to a conductivity of approx. 6 mS/cm and a pH value of 5.5. The solution was applied to a Heparin-Affinity-Resin column (Heparin immobilized on Toyopearl HW-65 ). The column was washed with low salt buffer solution (25 mmol/L tri-sodium citrate) and subsequently the complement Factor H was eluted by an ascending NaCl gradient (0-1 mol/L) in the same buffer.

**Table 12: Purification of complement Factor H by a combination of anion exchange chromatography, hydrophobic interaction chromatography pasteurization and a final heparin affinity chromatography**

| Sample | Absorption 280 nm | Factor H [mg/L] | Factor H [%] |
|---|---|---|---|
| Factor H from AEC + HIC + pasteurization | 3.0 | 3700 | 100 |
| Heparin affinity eluate | 6.2 | 8800 | 86 |

### b. Hydrophobic Interaction Chromatography (HIC)

A complement Factor H containing solution resulting from one of the methods or repetitions of the methods or combinations of the methods described under 1-4 can be further purified by hydrophobic interaction chromatography. Suitable ligands and/or media are referenced under 2 b.

Complement Factor H containing solutions are formulated to a corresponding conductivity of > 100 mS/cm (25 °C) and a pH of 5 - 9 in suitable buffer systems (e.g. saline or phosphate-buffer or citrate buffer). The so conditioned solution is then applied to a column packed with Butyl Sepharose FF (GE Healthcare ). The column is washed with high salt buffer solution (25 mmol/L tri-sodium citrate, 2 mol/L NaCl) and subsequently the complement Factor H is eluted by an descending NaCl gradient (2-0 mol/L) in the same buffer.

### c. Anion exchange chromatography (AEC)

A complement Factor H containing solution resulting from one of the methods or repetitions of the methods or combinations of the methods described under 1-4 can be further purified by anion exchange chromatography. Suitable ligands and/or media are referenced under 2 c.

Complement Factor H containing solutions are formulated to a corresponding conductivity of < 10 mS/cm (25 °C) and a pH of 6 - 9 in suitable buffer systems (e.g. saline or phosphate-buffer or citrate buffer). The so conditioned solution is then applied to a column packed with Q-Sepharose XL (GE Healthcare ). The column is washed with low salt buffer solution (25 mmol/L tri-sodium citrate) and subsequently the complement Factor H is eluted by an ascending NaCl gradient (0 - 1 mol/L) in the same buffer.

### d. Cation exchange chromatography (CEC)

A complement Factor H containing solution resulting from one of the methods or repetitions of the methods or combinations of the methods described under 1-4 can be further purified by cation exchange chromatography. Suitable ligands and/or media are referenced under 2 d.

Complement Factor H containing solutions are formulated to a corresponding conductivity of < 10 mS/cm (25 °C) and a pH of 5 - 8 in suitable buffer systems (e.g. saline or phosphate-buffer or citrate buffer). The so conditioned solution is then applied to a column packed with SP-Sepharose FF (GE Healthcare ). The column is washed with low salt buffer solution (25 mmol/L tri-sodium citrate) and subsequently the complement Factor H is eluted by an ascending NaCl gradient (0 - 1 mol/L) in the same buffer.

### e. Hydroxyapatite chromatography (HAC)

A complement Factor H containing solution resulting from one of the methods or repetitions of the methods or combinations of the methods described under 1-4 can be further purified by hydroxyapatite chromatography. Suitable ligands and/or media are referenced under 2 e.

The source fraction (complement Factor H rich wash from Heparin Affinity chromatography out of 8 % supernatant from a Cohn/Oncley fractionation, or redissolved Fraction III from a Cohn/Oncley fractionation, or redissolved Precipitate B from a Kistler/Nitschmann fractionation) is formulated to a corresponding conductivity of < 15 mS/cm (25 °C) and a pH of 5 - 9 in suitable buffer systems (e.g. saline or phosphate-buffer or citrate buffer). The so conditioned solution is then applied to a column packed with HA Ultrogel (Pall ). The column is washed with low salt buffer solution (e.g. 10 mmol/L potassium phosphate) and subsequently the complement Factor H is eluted by an ascending phosphate gradient (0 -1 mol/L).

### f. Immunoaffinity chromatography

A complement Factor H containing solution resulting from one of the methods under 1 -4 can be further purified by immunoaffinity chromatography. Suitable ligands and/or media are referenced under 2 f.

### 7. Complement Factor H formulations

In order to obtain chemically and physically stable therapeutic complement Factor H preparations, in such a way that these preparations can be stored at e.g. 2-8 °C or above and handled reconstituted and used at ambient room temperature (e.g. 18-28 °C), purified (according to 1.-5.) complement Factor H can be formulated together with suitable excipients and stabilizers and subsequently lyophilized.

Suitable excipients and stabilizers can be any substance or combinations of substances from the following groups:
Bulking agents
Cryoprotectants
Lyoprotectants
Tonicity modifiers
Surfactants/detergents
Buffer
Antioxidants

### a. Bulking agents

The term "bulking agents" as used herein, is intended to designate agents which provide or contribute to the structure of the freeze-dried product, in other words a visually good looking lyophilized cake or a pharmaceutically elegant product and, optionally, contributes to stabilization of the active substance.
Typical examples for substances used as bulking agents are e.g. mannitol, glycine, sucrose, lactose. Typical concentrates are in the range of 0-10 % w/v.

### b. Cryoprotectants

The term "cryoprotectants" as used herein, is intended to designate agents which protect the active substance from freezing-induced stress or damage.
Typical examples for substances used as cryoprotectants are e.g. polyols (mannitol), saccharides (sucrose, trehalose),surfactants (polysorbate, polyoxamer, polyethylene glycol). Typical concentrations are in the range of 0 -10 % w/v.

### c. Lyoprotectants

The term "lyoprotectants" as used herein, is intended to designate agents which protect the active substance from dehydration induced stress or damage (e.g. due to partially removing the hydration shell of a protein) during lyophilization.
Typical examples for substances used as lyoprotectants are e.g. sucrose, trehalose, sodium glutamate, glycine, isoleucine, lysine hydrochloride, phenylalanine, and arginine. Typical concentrations are in the range of 0 - 10 % w/v.

### d. Tonicity modifiers

The term "tonicity modifiers" as used herein, is intended to designate agents which effect that the tonicity, i.e., osmolality of the preparation, is in the range of normal physiological fluids (e.g. blood or peritoneal fluid) in order to prevent post-administration reactions due to differential ion concentrations between the preparation and physiological fluids.
Typical examples for substances used as tonicity modifiers are e.g. NaCl, dextrose, glycerin, calcium chloride, potassium chloride.

### e. Surfactants/detergents

The term "surfactants/detergents" as used herein, is intended to designate agents which protect the active substance from air-to-solution interface or solution-to-surface interface induced stress or damage.
Typical examples for surfactants/detergents are e.g. poloxamers (e.g. poloxamer 188), polysorbates (e.g. Tween 20, Tween 80), polyoxyethylene lauryl ethers (e.g. Brij 35)
Typical concentrations range from about 0.001% to about 0.5%,

### f. Buffer

The term "buffer" as used herein, is intended to designate agents which maintain the pH of the preparation in a range where administration of the preparation (e.g. intravenous or subcutaneous) is normally well tolerated (e.g. pH 5 - 9) and, furthermore, avoid pH shifts and protect the active substance from pH induced stress or damage during lyophilization.
Typical examples for substances used as buffer substances are e.g. carbonate, phosphate, citrate, borate, trimethamine [(2-amino-2-hydroxymethyl-1,-3-propanediol),TRIS], glycine.

### g Antioxidants

The term "antioxidants" as used herein, is intended to designate agents that provide at least some reduction in oxidative stress or degradation of the active substance
Typical examples for substances used as antioxidants are e.g.g monothioglycerol, glutathione, citric acid, ascorbic acid, sodium metabisulfite, and sodium sulfite Typical concentrations are in the range from about 0.1 to about 1% (w/v).

**Table 13: Composition of formulations:**

| | |
|---|---|
| 1. | 0.85 % NaCl, 25 mmol/L tri-sodium citrate dihydrate pH 5-9, 1 % w/v glycine, 2 % sodium glutamate, 1 % arginine, 1 % isoleucine |
| 2. | 25 mmol/L tri-sodium citrate dihydrate pH 5-9, 1 % w/v glycine, 2 % sodium glutamate, 1 % arginine, 1 % isoleucine |
| 3. | 0.85 % NaCl, 25 mmol/L tri-sodium citrate dihydrate pH 5-9, 1 % w/v glycine, 2 % sucrose, 1 % arginine, 1 % isoleucine |
| 4. | 0.85 % NaCl. 25 mmol/L tri-sodium citrate dihydrate pH 5-9, 1 % w/v glycine, 2 % trehalose, 1 % arginine, 1 % isoleucine |
| 5. | 25 mmol/L tri-sodium citrate dihydrate pH 5-9, 1 % w/v glycine, 2 % sucrose, 1 % arginine, 1 % isoleucine |
| 6. | 25 mmol/L tri-sodium citrate dihydrate pH 5-9, 1 % w/v glycine, 2 % trehalose, 1% arginine, 1 % isoleucine |
| 7. | 0.85 % NaCl, 25 mmol/L tri-sodium citrate dihydrate pH 5-9, 1 % w/v glycine, 2 % sodium glutamate, 0.1 % Tween 80 |
| 8. | 0.85 % NaCl, 25 mmol/L tri-sodium citrate dihydrate pH 5-9, 1 % w/v glycine, 2 % sucrose, 0.1 % Tween 80 |
| 9. | 0.85 % NaCl, 25 mmol/L tri-sodium citrate dihydrate pH 5-9, 1 % w/v glycine, 2 % trehalose, 0.1 % Tween 80 |
| 10. | 25 mmol/L tri-sodium citrate dihydrate pH 5-9, 1 % w/v glycine, 2 % sodium glutamate, 0.1 % Tween 80 |
| 11. | 25 mmol/L tri-sodium citrate dihydrate pH 5-9, 1 % w/v glycine, 2 % sucrose, 0.1 % Tween 80 |
| 12. | 25 mmol/L tri-sodium citrate dihydrate pH 5-9, 1 % w/v glycine, 2 % trehalose, 0.1 % Tween 80 |

The solution comprising the purified complement Factor H according to the methods described in 1-5 is concentrated to the intended final concentration by means of ultrafiltration (e.g. TFF utilizing 100 kDa cut-off membranes) and diafiltered against 5-10 exchanges of a suitable buffer solution (e.g. 0.85 % NaCl, 25 mmol/L tri-sodium citrate dihydrate pH 5-9 ). Subsequently further excipients according to the examples in 6 a - g are added (e.g. 1 % w/v glycine, 2 % sucrose, 1 % arginine, 1 % isoleucine ) and completely dissolved. The formulated complement Factor H solution is then filtered through a sterilization grade filter as a prerequisite for the subsequent fill and finish procedures.

### 8. Complement Factor H preparations (Lyophilization)

In order to obtain chemically and physically stable therapeutic complement Factor H preparations as described in 6, the residual moisture content of the lyophilized product must be low enough to protect the active substance from degradation. A residual moisture content of ≤ 3 % can be achieved by suitable lyophilization procedures and should not be exceeded in this context.

The formulated complement Factor H preparation is filled in suitable vials for lyophilization. The vials are loaded into a suitable lyophilizer and the preparation is frozen at a shelf temperature of - 47 °C during 4-5 h (approx. 0.5-1 °C/min) at ambient pressure conditions. Subsequently, the chamber pressure is decreased to 0.15 mbar during 30-60min and the shelf temperature is raised to -10 °C during 30-60 min. accordingly. After 10-15 h , the shelf temperature is further raised to 5 °C and held constant for additional 10-15 h. Subsequently the shelf temperature is raised to 20 °C and held for 24-30 h. Typically, during this step, the product temperature reaches the shelf temperature after approx. 1/2 to 1/3 of the time. Secondary drying is then achieved by raising the shelf temperature to 35 °C and held constant for 24 - 30 h.

### 9. Example for a preferred embodiment

In a preferred embodiment, the 8 % supernatant (2400-2700 L) of a modified Cohn/Oncley fractionation is subjected to a Heparin-affinity chromatography. Approx. 0.08 L Heparin-Affinity-Resin (Heparin immobilized on Toyopearl HW-65) are added per liter of 8 % supernatant and the suspension is stirred for approx. 1 h in the cold. Subsequently, the resin is recovered by filtration and transferred into a chromatography column. Complement Factor H is eluted from the resin utilizing a solution containing approx. 0.25 mol/L tri-sodium citrate dihydrate at pH 6.25 whilst ATIII remains bound to the column.
The complement Factor H rich citrate-wash (400-700 L; 32 mS/cm, pH 6.2) is warmed up to 30 °C. A PEG 4000 concentrate (25 % w/v) is added to achieve a final PEG concentration of 7.5 % w/v. The mixture is stirred for 30 min. (30 °C) and filtered through a combination of depth filters following addition of 10 g/L perlite filter aid.
The complement Factor H containing PEG filtrate is formulated to a conductivity of 5-6 mS/cm (25 °C) and a pH of 9 in 25 mmol/L TRIS buffer. The so conditioned solution is then applied to a column packed with 80 L Q-Sepharose XL (GE Healthcare ). The column is washed with low salt buffer solution (25 mmol/L tri-sodium citrate) and subsequently the complement Factor H is eluted by a NaCl step gradient (0.3 mol/L) in the same buffer.

The complement Factor H rich AEC-eluate (100-300 L) is formulated to a conductivity of 135 mS/cm (25 °C) and a pH of 5.5 in citrate buffer. The so conditioned solution is then applied to a column packed with 80 L Butyl Sepharose FF (GE Healthcare ). The column is washed with high salt buffer solution (25 mmol/L tri-sodium citrate, 2 mol/L NaCl) and subsequently the complement Factor H is eluted by an descending NaCl gradient (2 - 0 mol/L) in the same buffer.

The HIC-purified complement Factor H (80-200 L) is formulated to 0.9 % NaCl, 50 mmol/L tri-sodium citrate dihydrate pH 5.5 at a protein concentration of Abs. 280 nm = 15 by means of tangential flow ultrafiltration (TF-UF) using membranes with a cut-off of 100 kDa. Per L of solution 1 kg of sucrose is added and dissolved. The pH value is adjusted to 5.5. Subsequently the stabilized solution is heated to 60 °C and held at that temperature for 10 h. Accordingly the solution is cooled to ambient temperature and diluted 1:3 with citrate buffer pH 5.5.

The diluted pasteurized complement Factor H containing solution (approx. 6 mS/cm; pH 5.5) is then applied to a Heparin-Affinity-Resin column (40 L Heparin immobilized on Toyopearl HW-65 ). The column is washed with low salt buffer solution (25 mmol/L tri-sodium citrate) and subsequently the complement Factor H is eluted by an ascending NaCl gradient (0 - 1 mol/L) in the same buffer.

Following Heparin affinity chromatography, the complement Factor H solution is filtered through an accepted virus retentive filter, Planova ™ 20 nm (Asahi corporation). Up to 100 L Purified complement Factor H solution are filtered through a 1 m² Planova 20 module following prefiltration through one ore more 10" (0.7 m² ) 0.1 µm PVDF filter cartridge(s) (e.g. Durapore, Millipore).
Subsequently, the purified complement Factor H solution is diafiltered and concentrated by TF-UF to a concentration of at least 50 mg/mL* in 25 mmol/L tri-sodium citrate pH 7 + 0.85 % NaCl. Excipients are added (1 % w/v glycine, 2 % sodium glutamate, 1 % arginine, 1 % isoleucine ) and the pH is adjusted to 8. The solution (2000-4000 mL) is filtered through a sterilization grade filter ( ≤ 0.22 µm ) and filled into the final containers (e.g. 30 mL injection vials glass type II, chlorobutyl rubber lyophilization stoppers ).

The filled vials are lyophilized accordingly, as described under 7.

* determined by utilizing a pure complement Factor H reference preparation as calibrator

**Table 14: Purity (as determined via size exclusion HPLC*) of intermediates and final formulated complement Factor H preparation obtained from large scale manufacturing**

| Sample | HMW impurities ¹⁾ | Factor H | LMW impurities ²⁾ |
|---|---|---|---|
| | [Area %] | [Area %] | [Area %] |
| PEG 4000 filtrate | 0.5 | 17.9 | 81.6 |
| AEC eluate | ---- | 53.1 | 46.9 |
| HIC eluate | 0.2 | 83.1 | 16.6 |
| Post Pasteurization | 6.0 | 84.6 | 9.4 |
| Heparin AFC eluate | 2.9 | 93.1 | 3.9 |
| Formulated bulk (approx. 50 mg/mL) | 2.9 | 95.7 | 1.4 |

| | | | |
|---|---|---|---|
| * TSK G3000 SW_{XL} ¹⁾ Retention time < Factor H ²⁾ Retention time > Factor H | | | |

## Claims

1. A method for purification of complement Factor H in a solution comprising the steps of:
a) providing a fraction comprising complement Factor H by large-scale fractional precipitation of human plasma or serum with ethanol, wherein at least one further commercially obtainable pharmaceutical protein is produced by said large-scale fractional precipitation process
b) further purifying complement Factor H by at least one purification method selected from the group:
I. Heparin affinity chromatography
II. Hydrophobic interaction chromatography (HIC)
III. Anion exchange chromatography (AEC)
IV. Cation exchange chromatography (CEC)
V. Hydroxyapatite chromatography (HAC)
VI. Immunoaffinity chromatography
c) treating the complement Factor H containing fraction at least once before, during or after step b) of said purification process with a virus/pathogen inactivation and/or elimination method;
wherein the purification of complement Factor H comprises the following process steps:
I. an initial anion exchange chromatography
II. followed by a hydrophobic interaction chromatography
III. followed by pasteurization
IV. followed by a heparin affinity chromatography
V. followed by nanofiltration.

2. Method according to claim 1, wherein the purification method of step b) is repeated one or several times or is combined with another purification method.

3. Method according to claim 1 or 2 wherein said other commercially obtainable pharmaceutical protein is selected from the group consisting of albumin, antithrombin III, Factor VIII, Factor IX, Fibrinogen, Factor XIII, thrombin (FIIa), FVII/IIa, prothrombin-complex preparations (PPSB), alpha-1 proteinase inhibitor, C1 Inhibitor, immunoglobulins, transferrin and butyrylcholinesterase.

4. Method according to claim 1 to 3 wherein the virus/pathogen inactivation or elimination method is pasteurisation, solvent/detergent treatment and/or nanofiltration.

5. Method according to claim 1 to 4 wherein the fraction comprising complement Factor H is the 8% ethanol supernatant (Fraction I in Cohn/Oncley fractionation) or Fraction III (Cohn/Oncley fractionation) or Precipitate B in Kistler/Nitschmann fractionation.

6. Method according to claim 1 to 5 wherein the purification process comprises additionally a delipidation step and/or the inhibition and/or removal of a protease.

7. A method according to claim 1 to 6 wherein the fraction comprising complement Factor H is a waste fraction.

## Patentansprüche

1. Verfahren zur Reinigung von Komplementfaktor H in einer Lösung, umfassend die Schritte:
a) Bereitstellen einer Fraktion, umfassend Komplementfaktor H, durch großtechnisch fraktionierte Fällung von menschlichem Plasma oder Serum mit Ethanol, wobei mindestens ein weiteres kommerziell erhältliches pharmazeutisches Protein durch diesen großtechnisch fraktionierten Fällungsprozess erzeugt wird,
b) ferner Reinigen von Komplementfaktor H durch mindestens ein Reinigungsverfahren, ausgewählt aus der Gruppe:
I. Heparinaffinitätschromatographie
II. hydrophobe Interaktionschromatographie (HIC)
III. Anionenaustauschchromatographie (AEC)
IV. Kationenaustauschchromatographie (CEC)
V. Hydroxyapatitchromatographie (HAC)
VI. Immunaffinitätschromatographie
c) Behandeln der Komplementfaktor H enthaltenden Fraktion mindestens einmal vor, während oder nach Schritt b) des Reinigungsprozesses mit einem Virus-/Pathogeninaktivierungs- und/oder -eliminierungsverfahren,
wobei die Reinigung von Komplementfaktor H die folgenden Prozessschritte umfasst:
I. eine anfängliche Anionenaustauschchromatographie
II. gefolgt von einer hydrophoben Interaktionschromatographie
III. gefolgt von Pasteurisierung
IV. gefolgt von einer Heparinaffinitätschromatographie
V. gefolgt von Nanofiltration.

2. Verfahren nach Anspruch 1, wobei das Reinigungsverfahren von Schritt b) ein- oder mehrmals wiederholt wird oder mit einem anderen Reinigungsverfahren kombiniert wird.

3. Verfahren nach Anspruch 1 oder 2, wobei das andere kommerziell erhältliche pharmazeutische Protein aus der Gruppe ausgewählt ist, bestehend aus Albumin, Antithrombin III, Faktor VIII, Faktor IX, Fibrinogen, Faktor XIII, Thrombin (FIIa), FVII/IIa, Prothrombinkomplexpräparaten (PPSB), alpha-1-Proteinaseinhibitor, C1-Inhibitor, Immunglobulinen, Transferrin und Butyrylcholinesterase.

4. Verfahren nach Anspruch 1 bis 3, wobei das Virus-/Pathogeninaktivierungs- oder -eliminierungsverfahren Pasteurisierung, Solvent/Detergent-Behandlung und/oder Nanofiltration ist.

5. Verfahren nach Anspruch 1 bis 4, wobei die Komplementfaktor H umfassende Fraktion der 8-%-Ethanolüberstand (Fraktion I bei der Cohn/Oncley-Fraktionierung) oder Fraktion III (Cohn/Oncley-Fraktionierung) oder Niederschlag B bei der Kistler/Nitschmann-Fraktionierung ist.

6. Verfahren nach Anspruch 1 bis 5, wobei der Reinigungsprozess außerdem einen Lipidentfernungsschritt und/oder die Inhibierung und/oder Entfernung einer Protease umfasst.

7. Verfahren nach Anspruch 1 bis 6, wobei die Komplementfaktor H umfassende Fraktion eine Abfallfraktion ist.

## Revendications

1. Procédé pour la purification du Facteur H du complément dans une solution comprenant les étapes consistant à :
a) fournir une fraction comprenant le Facteur H du complément par la précipitation fractionnelle à grande échelle de plasma ou sérum humain avec de l'éthanol, dans lequel au moins une autre protéine pharmaceutique pouvant être obtenue dans le commerce est produite par ledit processus de précipitation fractionnelle à grande échelle
b) purifier encore le Facteur H du complément par au moins un procédé de purification sélectionné dans le groupe constitué de :
I. la chromatographie d'affinité sur héparine
II. la chromatographie d'interaction hydrophobe (HIC)
III. la chromatographie d'échange d'anions (AEC)
IV. la chromatographie d'échange de cations (CEC)
V. la chromatographie d'hydroxyapatite (HAC)
VI. la chromatographie d'immunoaffinité
c) traiter la fraction contenant le Facteur H du complément au moins une fois avant, pendant ou après l'étape b) dudit processus de purification avec un procédé d'inactivation et/ou d'élimination de virus/pathogène ;
dans lequel la purification du Facteur H du complément comprend les étapes de processus suivantes :
I. une chromatographie d'échange d'anions initiaux
II. suivie d'une chromatographie d'interaction hydrophobe
III. suivie d'une pasteurisation
IV. suivie d'une chromatographie d'affinité sur héparine
V. suivie d'une nanofiltration.

2. Procédé selon la revendication 1 dans lequel le procédé de purification de l'étape b) est répété une ou plusieurs fois ou est combiné avec un autre procédé de purification.

3. Procédé selon la revendication 1 ou 2 dans lequel ladite autre protéine pharmaceutique pouvant être obtenue dans le commerce est sélectionnée dans le groupe constitué d'albumine, d'antithrombine III, de Facteur VIII, de Facteur IX, de Fibrinogène, de Facteur XIII, de thrombine (FIIa), de FVII/IIa, de préparations de complexe prothrombinique (PPSB), d'inhibiteur d'alpha-1 protéinase, d'inhibiteur C1, d'immunoglobulines, de transferrine et de butyrylcholinestérase.

4. Procédé selon les revendications 1 à 3 dans lequel le procédé d'inactivation ou d'élimination de virus/pathogène est la pasteurisation, le traitement par solvant/détergent et/ou la nanofiltration.

5. Procédé selon les revendications 1 à 4 dans lequel la fraction comprenant le Facteur H du complément est le surnageant à 8 % d'éthanol (Fraction I du fractionnement de Cohn/Oncley) ou la Fraction III (fractionnement de Cohn/Oncley) ou le fractionnement par précipité B par Kistler/Nitschmann.

6. Procédé selon les revendications 1 à 5 dans lequel le processus de purification comprend en plus une étape de dégraissage et/ou l'inhibition et/ou le retrait d'une protéase.

7. Procédé selon les revendications 1 à 6 dans lequel la fraction comprenant le Facteur H du complément est une fraction de déchet.
